# EUROPEAN PATENT SPECIFICATION

(11) **EP 3 413 871 B1**
(45) Date of publication and mention of the grant of the patent: **06.11.2019**
(21) Application number: 17705172.9
(22) Date of filing: 02.02.2017
(51) Int. Cl.: A61K 9/00, A61K 31/7032, A61K 31/728, A61P 27/02, A61P 31/04, A61K 45/06, A61K 47/12, A61K 47/36, A61K 9/08

(54) **COMPOSITION FOR THE USE IN THE TREATMENT OF BACTERIAL INFECTIONS**
ZUSAMMENSETZUNG ZUR VERWENDUNG BEI DER BEHANDLUNG BAKTERIELLER INFEKTIONEN
COMPOSITION POUR UTILISATION DANS LE TRAITEMENT D'INFECTIONS BACTÉRIENNES

(30) Priority: 08.02.2016 IT UB20160543; 08.11.2016 WO PCT/IB2016/056719
(43) Date of publication of application: 19.12.2018
(73) Proprietor: Sooft Italia S.p.A., 63833 Montegiorgio (FM) (IT)
(72) Inventor: BIONDI, Marco, I-63822 Porto San Giorgio (FM) (IT); BIONDI, Piero, I-63822 Porto San Giorgio (FM) (IT); STAGNI, Edoardo, 00165 Roma (IT); STAGNI, Marcello, 63822 Porto San Giorgio (FM) (IT)
(74) Representative: Sarpi, Maurizio
(86) International application number: PCT/IB2017/050561
(87) International publication number: WO 2017/137871

(56) References cited:
- EP-A2- 2 494 954
- CN-A- 104 095 869
- JP-A- 2006 104 164
- US-A1- 2009 111 770

## Description

### Technical field of the invention

The present invention relates to the medical pharmaceutical field and, more specifically, the technical field of topical preparations and used in the treatment of bacterial infections. Although the composition of the present invention and its use in the treatment of microbial infections is preferably directed toward ophthalmic applications directly onto corneal surface, it is contemplated that the present invention would also apply to other forms of topical delivery, as well as for oral and parenteral administration.

### Background of the invention

Although ocular infection may be considered to be minor infection, it can be vision-threatening and must be treated effectively by appropriate and safe use of suitable agents capable of acting against infection, either by inhibiting the spread of an infectious agent or by killing the infectious agent outright. Ocular bacterial infections affect subjects of all ages and with high frequency newborns and children.

The human eye, as all other structures of the body that are in contact with the external environment, is often subject to attack by pathogenic microorganisms, such as bacteria, viruses, protozoa, fungi etc., responsible of more or less important and extensive infections.

Ocular infections can affect the external structures of the eye, for example the sebaceous glands located in the eyelids (chalazion), an eyelid gland (stye), the edge of the eyelid and conjunctiva (blepharitis or blepharoconjunctivitis), infections can involve the lacrimal ducts, more precisely the lacrimal sac and/or lacrimal grooves (dacryocystitis and/or canaliculitis); the conjunctival mucosa (conjunctivitis); when the infection affects the cornea keratitis or keratoconjunctivitis attains.

Ocular infections affecting the posterior part of the eyeball cause uveitis if the process involves the vascular membrane of the eye, whereas endophthalmitis are severe infectious process located within the eyeball.

Symptoms of eye infections are clearly closely linked to the structures involved and the degree of extension of the disease process. The first occurring symptoms are: more or less evident swelling eyelid, conjunctival hyperemia (redness of the conjunctiva), burning, tearing or secretion and photophobia (intolerance to light). In the more severe cases the above symptoms are associated with ocular pain and hypertonicity (increased eye pressure).

Most cases of acute ocular bacterial infections resolve spontaneously within 7-10 days, but antibiotic administration can decrease disease severity, transmission and also minimize the complication and reinfection rates. The therapeutic treatment for ocular bacterial infections requires the use of eye drops and ophthalmic ointments having antimicrobial activity to be applied directly in the eye.

The respective advantages of eye drops and ointments include preserved visual acuity and prolonged contact, and a soothing effect.

Many different antibiotic drugs have been included in formulation designed for topical administration, and in particular for topical ophthalmic administration. Practice patterns for prescribing topical antibiotics vary. Most practitioners prescribe a broad-spectrum agent on an empirical basis without cultural assay for a routine, mild-to-moderate case of bacterial conjunctivitis, and instruct patients to seek follow-up care if the expected improvement does not occur or if vision becomes affected.

Thus, generally, broad-spectrum antibiotics, active against most of the bacteria, are prescribed; when the germ responsible for the infection is isolated, eye drops or ophthalmic ointment containing a specific drug are preferred.

Sodium sulfacetamide, chloramphenicol, gentamicin, tobramycin, azithromycin, neomycin, trimethoprim and polymyxin B combination, ciprofloxacin, ofloxacin, gatifloxacin, and erythromycin are representatives of commonly used first-line agents.

As a support to antibiotic therapy, often a complementary use of corticosteroid eye drops having anti-inflammatory action is recommended.

For example, for the treatment of ocular infection supported by *Pseudomonas aeruginosa* the most indicated eye drops is formulated with gentamicin; fusidic acid is instead recommended for bacterial conjunctivitis caused by staphilococci.

Particularly challenging is the treatment of the bacterial keratitis forms, as a vision-threatening process and a major cause of corneal opacity and loss of vision worldwide. Bacterial keratitis is a potentially devastating ocular infection that may occur when the corneal epithelial barrier is compromised due to injury or trauma, leading to ulceration and infiltration of inflammatory cells. A particular feature of bacterial keratitis is its rapid progression; corneal destruction may be complete in 24-48 hours with some of the more virulent bacteria. In fact, corneal ulceration, stromal abscess formation, surrounding corneal edema, and anterior segment inflammation are characteristic of this disease. Infection is largely due to Gram-positive *S*. *aureus, S. epidermidis,* and several *Streptococcus* and *Bacillus* spp., but also Gram-negative bacteria such as *P. aeruginosa, S. marcescens, Moraxella lacunata, Microbacterium liquefaciens,* and *H. influenzae.* Contact lenses are increasingly involved in keratitis: contact lens wearing now accounts for more than one half of all cases of bacterial keratitis and has become the most important risk factor.

Immediate diagnosis and treatment are important to avoid vision-threatening outcomes, including corneal scarring or perforation. The choice of treatment is based on the use of a single antimicrobial, or a combination of antimicrobials, that provides a broad range of activity against both Gram-positive and Gram-negative bacteria. Once the pathogen has been isolated, treatment can be modified according to the *in vitro* susceptibility pattern of the isolate. Specially prepared fortified combined antibiotics have been used to provide broad-spectrum coverage. The limited availability, cost, adverse effects, and inconvenience of these fortified preparations have led to the use of commercially topical fluoroquinolones. Consequently, emerging resistance of *Staphylococcus aureus* to ciprofloxacin and ofloxacin has raised concern over the use of monotherapy with these agents for suspected cases of bacterial keratitis. This is especially true given the high rates of microbial keratitis caused by Gram-positive organisms. Nevertheless, fortified topical antibiotics and fluoroquinolones are still the mainstay of bacterial keratitis therapy.

Antimicrobial therapy is also administrated in perioperative prophylaxis in ocular surgery.

Cataract surgery has become one of the most prevalent surgical procedures due to changes in population structure and increasing life expectancy. As a result of technical progress, it is now a very safe and predictable procedure, but endophthalmitis is still one of the most serious complications of cataract surgery. The risk of endophthalmitis reported in prospective studies is 0.38% in Europe. The microorganism responsible for endophthalmitis may be derived from conjunctival flora, contaminated instruments, irrigating solutions, the implant, or airborne contamination. The best means of prevention of endophthalmitis is therefore compliance with strict surgical hygiene. The organisms most commonly isolated from endophthalmitis occurring after cataract surgery are Gram-positive bacteria (86.7% of cases), including *Staphylococcus epidermidis, Staphylococcus aureus* and *Streptococcus pneumoniae,* while Gram-negative bacteria represent 13.3% of cases.

A variety of practices and procedures have been proposed for a long time in order to minimize the incidence of postoperative infection. Two approaches to prophylaxis have been proposed. One consists of reducing the number of organisms on the surface of the eye by using topically applied antisepsis and/or antibiotics. The other involves diffusion of antibiotics into the ocular tissues during the perioperative period via the topical, subconjunctival, systemic, or intracameral routes.

Several clinical trials show that preoperative topical antibiotic prophylaxis reduces the number of bacteria in the conjunctival sac versus placebo. The use of postoperative topical antibiotics for up to two weeks is recommended to minimize the risk of infection, but their usefulness has never been formally demonstrated. Moreover, the long treatment period and the excessive use of antibiotics play a role in the development of resistant bacteria species.

When minor ocular trauma and injuries consisting of trivial superficial corneal epithelial abrasions occurs, generally a period of several hours to several days may be required before onset of infection and development of true corneal ulcers. Upadhyay considered that this interval could constitute a "window of opportunity" to prevent the development of corneal ulcer [Upadhyay M.P. et al. The Bhaktapur eye study: ocular trauma and antibiotic prophylaxis for the prevention of corneal ulceration in Nepal. Br. J Ophthalmol. 2001; 85:388-92]. The study demonstrated that application of antibiotic in an eye within 18 hours after a traumatic corneal abrasion prevents the subsequent development of suppurative ulceration in the treated cornea.

Furthermore, prophylaxis of ophthalmia neonatorum is a major element in the worldwide effort to prevent blindness. Neonatal conjunctivitis has decreased considerably in developed countries as a result of prophylaxis. However, it remains a major concern in developing countries. The bacteria commonly responsible for ophthalmia neonatorum are *Neisseria gonorrhoea* and *Chlamydia trachomatis* transmitted by the mother's genital tract and which require aggressive treatment. Gonococcal infection affects 0.4 infants per 1,000 births. The range of available eye drop preparations has recently changed in many countries. In the past, silver nitrate was routinely used to prevent infection, but this agent can cause burns of the newborn's eyes. Erythromycin is now more commonly used, but is not available in all countries.

However, beside the undoubtedly great benefit derived from the use of classical antibiotic active principle, side effects, toxicity and resistance issues related to the use of the most commonly used antibiotics are well known and such as to generate concerns, they have to be carefully considered and overcome. Growing resistance has been observed among ocular bacteria as with other systemic pathogens. The factors contributing to the development of drug resistance include overuse of antibiotics for systemic infection, as well as overuse of topical antibiotics in the eye. Reserving certain antibiotics for hospital use to avoid resistance to all antibiotics, therefore, appears to be a major issue to maintain the efficacy of certain "emergency" antibiotics.

An increasing number of guidelines recommend avoiding the use of fluoroquinolones and their combinations except for the most severe infections or following treatment failure [Simon C., Stille W. Therapie wichtiger infektionen. Wahl des Antibiotikums. In: Simon C, Stille W, editors. Antibiotika-Therapie in Klinik und Praxis. Stuttgart, Germany: Schattauer; 2000. pp. 363-6; Afssaps. Recommandations. Collyres et autres topiques antibiotiques dans les infections oculaires superficielles. Recommendations. Eyedrops and other topical antibiotics in superficial ocular infections. 2004: 1-6]. This strategy is designed to preserve the efficacy of these antibiotics by limiting their potential selective pressure due to their high frequency of mutation. Only a few of the registered antimicrobials are therefore recommended as first-line therapy. Several of them (i.e., rifampicin, aminoglycosides, fusidic acid) have a much higher frequency of mutation in pathogens than azithromycin. Furthermore, most of the currently available antimicrobials require at least 5 to 10 days of treatment, or, in few cases, a very high concentration single dose. Improved compliance is a key issue for antibiotic therapy, as it would ensure improved efficacy and, above all, less resistance. Patent document JP2006104164 discloses antibacterial activity of lactobionic acid.

A particular need, therefore, for effective topical composition having antibiotic activity, with low toxicity and minor risk to induce resistance exists. Such need is met by the invention now described, as will be apparent from description and figures of the present invention below.

### Summary of the invention

It is therefore an object of the present invention a composition comprising 4% w/w lactobionic acid, or a salt thereof, and one or more pharmaceutically acceptable excipients and carriers for the use in the treatment of bacterial eye infections. According to the invention the ophthalmic composition for the use in the treatment of bacterial eye infections further comprises hyaluronic acid, or a salt thereof.

The composition for the use in the treatment of bacterial eye infections is preferably directed toward ophthalmic applications, but it is contemplated that the present invention would also apply to other forms of topical delivery, as well as for oral and parenteral administration. A further object of the invention relates to an eye pad or ocular bandage impregnated with a composition comprising 4.0% w/w lactobionic acid, or a salt thereof.

### Brief description of the drawings

Figure 1A shows graphically the growth of scalar dilutions of a bacterial culture of *Staphylococcus aureus* in the presence and in the absence of a composition comprising 4% sodium lactobionate.
Figure 1B shows graphically the growth of scalar dilutions of a bacterial culture of *Staphylococcus xylosus* in the presence and in the absence of a composition comprising 4% sodium lactobionate.
Figure 1C shows graphically the growth of scalar dilutions of a bacterial culture of *Pseudomonas fluorescence* in the presence and in the absence of a composition comprising 4% sodium lactobionate.
Figure 1D shows graphically the growth of scalar dilution of a bacterial culture of *Corynebacterium macginleyi* in the presence and in the absence of a composition comprising 4% sodium lactobionate.
Figure 1E shows graphically the growth of scalar dilutions of a bacterial culture of *Escherichia coli* in the presence and in the absence of a composition comprising 4% sodium lactobionate.
Figure 2A shows the comparison of growth curves of *Staphylococcus aureus* cultures in the presence of compositions comprising sodium lactobionate and/or hyaluronic acid at different concentrations.
Figure 2B shows the comparison of selected growth curves of *Staphylococcus aureus* cultures from Figure 2A in the presence of composition comprising sodium lactobionate at different concentrations (1%, 2%, 4%).
Figure 2C shows the comparison of selected growth curves of *Staphylococcus aureus* cultures from Figure 2A in the presence of composition comprising hyaluronic acid at different concentrations (0.037%, 0.075%, 0.15%).
Figure 2D shows the comparison of selected growth curves of *Staphylococcus aureus* cultures from Figure 2A in the presence of compositions comprising the association of sodium lactobionate and hyaluronic acid at different concentrations (respectively 1% and 0.037%; 2% and 0.075%, 4% and 0.15%).
Figure 2E shows the comparison of selected growth curves of a *Staphylococcus aureus* culture from Figure 2A in the presence of composition comprising the association of sodium lactobionate and hyaluronic acid (respectively 4% and 0.15%), a composition comprising 4% sodium lactobionate, and a composition comprising 0.15% hyaluronic acid.

### Description of the invention

The purpose of the present invention is to provide a pharmaceutical composition for the use in the treatment of bacterial eye infections, whose fundamental and characteristic component is lactobionic acid, or a salt thereof.

In a particularly preferred embodiment of the invention the fundamental and characteristic component of the pharmaceutical composition is lactobionic acid, or a salt thereof, in association with hyaluronic acid, or a salt thereof.

As above mentioned, in a particularly preferred embodiment of the invention the composition according to the invention is formulated for ophthalmic topical administration, however it also applies to oher forms of topical delivery, as well as for oral and parenteral administration.

Lactobionic acid is a disaccharide formed by gluconic acid and galactose. As an acid, lactobionic acid can form salts with mineral cations such as calcium, potassium, sodium and zinc. Calcium lactobionate is a food additive used as a stabilizer. Potassium lactobionate is added to organ preservation solutions to provide osmotic support and prevent cell swelling. Mineral salts of lactobionic acid are also used for mineral supplementation. Lactobionic acid is also used in the cosmetics industry as an antioxidant and in the pharmaceutical industry as an excipient for formulation.

Lactobionic acid, has strong humectants and moisturizing properties, it stimulates the function of the barrier constituted by the corneal layer of the epidermis, and therefore stimulates the protective function of the stratum corneum, increasing the skin resistance to the chemical insult.

In the Italian patent n. 1404752, granted to the Applicant, for the first time the use of lactobionic acid in an eye drop for the treatment of corneal edema, also in association to inflammation phenomena, has been disclosed. Said eye drops is characterized by the presence of lactobionic acid in a concentration varying from 5% w/w to 20% w/w, and more preferably from 6% w/w to 16% w/w; in addition to lactobionic acid, the solutions according to the invention may further comprise other hyperosmotic agents, such as mannitol or sodium chloride, alone or in combination. Hyaluronic acid is a glycosaminoglycan, or a molecule formed by long, unbranched chains of disaccharide units with alternating glucuronic acid and N-acetylglucosamine, used in many fields:
in cosmetics, in the formulation of moisturizing, anti-aging and anti-wrinkle creams;
in aesthetic medicine, in the form of injectable filler to correct minor skin imperfections, such as wrinkles, thin lips, emptied breasts, scarring, etc;
in orthopaedics, hyaluronic acid is injected by intra-articular route (infiltration) for the treatment of arthrosis;
in the healthy field, the molecule is used for the preparation of oral anti-aging supplements; and
in ophthalmology, the hyaluronic acid is used for the preparation of eye drops or ophthalmic ointments for promoting healing of the conjunctiva following traumas.

Ophthalmic ointments and eye drops prepared with hyaluronic acid are useful in the treatment of all ocular disorders characterized by apparent dryness (dry eye or keratoconjunctivitis sicca) or by conjunctival lesions (e.g. traumatic conjunctivitis). In these cases, hyaluronic acid promotes the healing of the conjunctiva in relatively short time and, attenuating the symptoms, keeps the eye hydrated.

The Italian patent application N. 102016012593 and the international patent application PCT/IB2016/056719, whose this application claims priority, disclose an isotonic ophthalmic composition characterized by the association of lactobionic acid and hyaluronic acid, or their respective salts, wherein particularly preferred are their sodium salts, and their use in the treatment of alterations of the corneal-conjunctival surface due to inflammatory states, oxidative stress, unbalance of the redox potential and accumulation of free radicals, dry eyes syndrome and traumatic abrasions, as well as in the wound-healing and re-epithelisation of the corneal-conjunctival surface.

Further and more detailed studies carried out by the Applicant in relation to the specific formulation have confirmed the antimicrobial activity thereof which support its feasible application in the treatment of microbial infections.

According to the invention the composition for the use in the treatment of bacterial eye infections comprises lactobionic acid, or a salt thereof, at a concentration of 4.0% w/w.

According to the invention the composition for the use in the treatment of bacterial eye infections further comprises hyaluronic acid, or a salt thereof. In the embodiment comprising the association of lactobionic acid, or a salt thereof, and hyaluronic acid, or a salt thereof, the concentration of the latter varies between 0.01% w/w and 1.0% w/w, preferably between 0.05% w/w and 0.5% w/w, and more preferably the hyaluronic acid concentration is 0.15% w/w.

In a particular embodiment of the invention the composition as described hereinabove further comprises a buffering agent and/or and agent for adjusting osmolality in amounts whereby the composition is isotonic and has a physiologically acceptable pH. Accordingly, the composition of the invention is an aqueous solution, clear and colourless, with pH between 7.0 and 7.4, and isotonic osmolality between 250 and 350 mOsm/Kg.

Various buffering systems known to the skilled of the field can be employed in some embodiments of the present invention. However, in some preferred embodiments of the present invention the buffering system of sodium phosphate monobasic/dibasic is used.

The isotonicity of the composition is ensured by the presence of an adequate amount of lactobionic acid, or its salts, and in the embodiments that so provides, by the presence of an adequate amount of lactobionic acid and of hyaluronic acid, or their salts. Particularly preferred are sodium salts of the two acids.

In addition to the association of lactobionic acid and hyaluronic acid, or their salts, the composition of the present invention may further comprise stabilizing agents of the solution, lubricating agents, moisturizing agents such as hydroxypropylmethylcellulose, or structural or functional analogues.

According to the invention the composition also comprises preservatives and chelating agents. Among the preservatives and chelating agents, particularly preferred are disodium EDTA and N-hydroxymethylglycinate.

According to the invention the composition may include in its formulation also one or more additional therapeutic agents selected from the group of growth factors, anti-inflammatory agents, vitamins, fibronectin, collagen and amino acids.

According to the present invention the composition can be prepared in any pharmaceutical form that permits its topical administration, and in particularly preferred embodiments it is prepared in a pharmaceutically form that enables the topical administration in the conjunctival sac, such as eye drops, collirium, gels, ointments, spray, but preferably it is prepared in the form of eye drops, administrable as drops, each drop constituting a form of dosage unit so as to allow easy administration and uniformity of dosage. For illustrative purposes two examples of particularly preferred formulation are presented.

**Example 1 - Formulation**

| Ingredient | % w/w |
|---|---|
| Sodium lactobionate | 4.0 |
| Sodium dihydrogen phosphate dihydrate | 0.225 |
| Disodium phosphate dehydrate | 0.686 |
| Disodium EDTA | 0.100 |
| Sodium Hydroxymethylglycinate | 0.002 |
| Distilled water | q.s to 100 g. |

**Example 2 - Formulation**

| Ingredient | % w/w |
|---|---|
| Sodium lactobionate | 4.0 |
| Sodium hyaluronate | 0.15 |
| Sodium dihydrogen phosphate dihydrate | 0.225 |
| Disodium phosphate dehydrate | 0.686 |
| Disodium EDTA | 0.100 |
| Sodium Hydroxymethylglycinate | 0.002 |
| Distilled water | q.s. to 100 g. |

According to the invention the provided composition is particularly simple and highly tolerable by patients, as it comprises a very limited number of components and excipients; this represents an advantageous feature allowing to reduce to a minimum the risk of possible allergic or sensitization phenomena.

In particular, in the embodiment of the single-dose formulation or preservative-free multi-dose formulation, the preservative system can be eliminated.

**Example 3 - Single-dose or preservative-free multi-dose formulation**

| Ingredient | % w/w |
|---|---|
| Sodium lactobionate | 4.0 |
| Sodium dihydrogen phosphate dihydrate | 0.225 |
| Disodium phosphate dihydrate | 0.686 |
| Distilled water | q.s. to 100 g |

**Example 4 - Single-dose or preservative-free multi-dose formulation**

| Ingredient | % w/w |
|---|---|
| Sodium lactobionate | 4.0 |
| Sodium hyaluronate | 0.15 |
| Sodium dihydrogen phosphate dihydrate | 0.225 |
| Disodium phosphate dihydrate | 0.686 |
| Distilled water | q.s. to 100 g |

In an particular embodiment of the invention the ophthalmic topic use of the composition can include also the impregnation of sterile eye pad or ocular bandage for topically dispensing the composition on the eye surface. Such gauze pads, in a sterile package, perform a detergent, disinfectant, decongestant and refreshing action and in addition ensure a perfect cleaning, reduce the swelling of the eyelids, act as decongestants and anti-inflammatories. Therefore, it is another object of the invention the composition comprising lactobionic acid, or a salt thereof, or comprising the association of lactobionic acid, or a salt thereof, and hyaluronic acid, or a salt thereof, impregnated eye pads and ocular bandages according to the appended claims. Disclosed herein is a method of treating a microbial infection, in particular a method of treating a bacterial infection in the eye of a subject, comprising ophthalmically administering a therapeutically effective dose of the pharmaceutical composition, as described above. Infective diseases of the eye for which the compositions for use of the invention are useful include without limitation conjunctivitis, keratitis, blepharitis, blepharoconjunctivitis, orbital and preseptal cellulitis and endophthahnitis. In preferred embodiments the infected tissue is one that is directly bathed by the lacrimal fluid, as in conjunctivitis, keratitis, blepharitis and blepharoconjunctivitis.

By the term "treatment", as used in the present description, a reduction in severity and/or frequency of symptoms, elimination of symptoms and/or underlying causes, prevention of the occurrence of symptoms and/or of their causes, improvement of, or remedy to, the injury caused by the infection is meant. Thus according to the invention the compositions for the treatment allow the prevention of occurrence of bacterial eye infections in a predisposed individual and the treatment of the symptoms in a clinically symptomatic individual. In infective diseases of the eye where the causal organism is non-bacterial, there can be benefit in prophylactic use of a composition of the invention to control secondary bacterial infections. Examples of such situations include conjunctivitis and keratitis of viral etiology, e.g., adenoviral conjunctivitis, molluscum contagiosum, herpes simplex conjunctivitis and keratitis, etc., and fungal keratitis. Prophylactic uses of a composition of the invention also include post-traumatic prophylaxis, especially post-surgical prophylaxis, and prophylaxis prior to ocular surgery.

The use of the present invention is not limited to humans, but can be extended, where appropriate, to other mammals.

The composition for use according to the invention administered in a therapeutically effective amount.

With the expression "therapeutically effective amount" a non-toxic amount, sufficient to provide the desired effect, i.e., the prevention or the treatment of microbial infection as above mentioned is meant. Comprehensibly, the "therapeutically effective amount" will vary from subject to subject, depending on the general conditions thereof, modes of administration, and the like. Therefore, it is not always possible to specify the exact extent of the "therapeutically effective amount."

The amount of a "therapeutically effective dose for The amount of a "therapeutically effective dose for treatment and/or prophylaxis of a bacterial infection" depends, among other factors, on the particular species, age and body weight of the subject in the need of the treatment; the particular condition for which treatment or prophylaxis is sought; and the severity of the condition.

In a particularly preferred embodiment of the invention, usually 1-2 drops of the composition are instilled into the conjunctival sac, 2-3 times a day or more, however, the therapeutically effective amount is defined by the physician as a function of patient condition. The composition can also be instilled while wearing contact lenses. Dosage and method of administration will be adjusted to provide sufficient levels of the active ingredient or to maintain the desired effect. Additional factors that should be considered include: severity of the condition, course of the disease, age, weight and gender of the patient, time and frequency of administration, diet, association with other drugs, sensitivity reactions, tolerance and response to therapy.

The composition according to the present invention has proved to be highly tolerable by patients. According to the invention the provided composition is particularly simple, as it comprises a very limited number of components and excipients: this represents an advantageous feature that allows to reduce to a minimum the risk of possible allergic or sensitization phenomena.

In particular, in the embodiment of the single-dose formulation or preservative-free multi-dose formulation, the preservative system can be eliminated.

The invention and its accomplishment will be now described through non limitative examples which will enable the skilled persons to fully understand the advantages deriving from the use thereof.

### EXPERIMENTAL PART

### Evaluation of in vitro antimicrobial activity of the composition according to the invention comprising 4% sodium lactobionate

The antimicrobial activity of a composition comprising 4% sodium lactobionate was assayed on scalar concentration (1x10² - 1x10⁶ CFU/ml) of bacterial cultures of different speciess, some of them being ocular and conjunctiva specific pathogens, such as *Staphylococcus aureus* and *Corynebacterium macginleyi,* and other responsible, and other such as *Pneumonas fluorescens* which is an unusual cause of disease in humans, and usually affects patients with compromised immune systems (e.g., patients on cancer treatment), *Staphylococcus xylosus,* generally not considered a human pathogen, and *Escherichia coli.*

Bacterial cultures were grown in the appropriate medium for 24 hours in the presence and in the absence of the tested composition. The bacteria were suspended in a sterile physiological saline solution and bacterial cell density was assessed by optical density (OD) at 620 nm (Biotek Instruments, Elx-800).

### Results

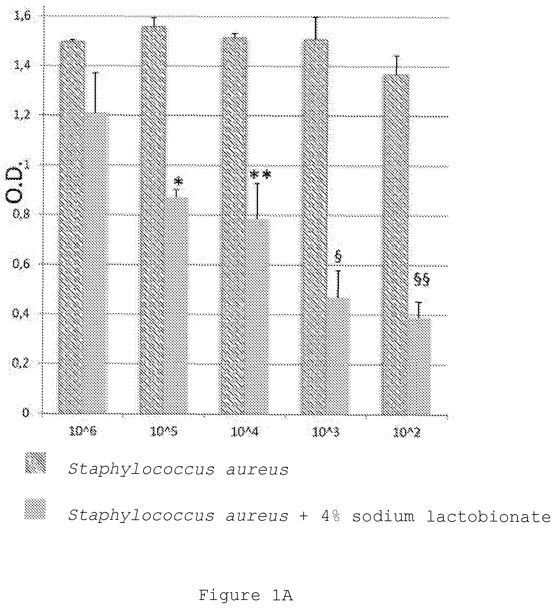

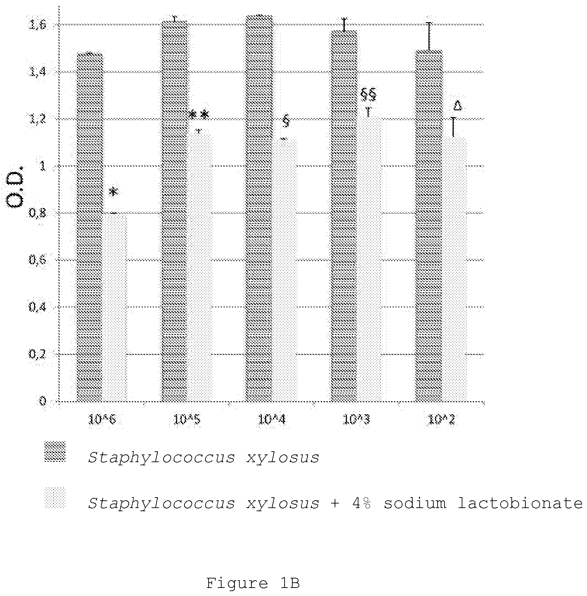

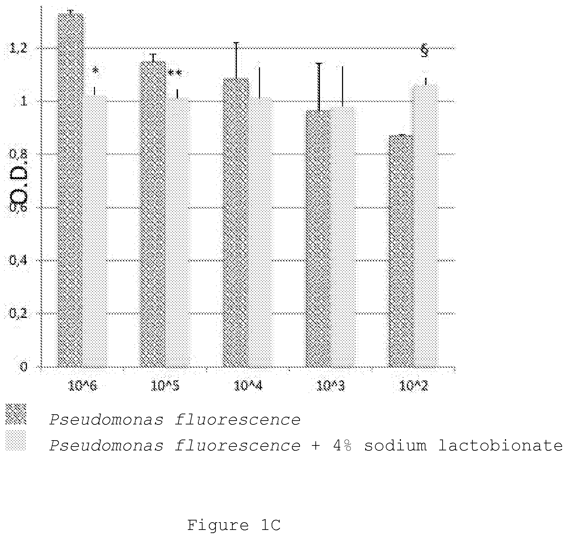

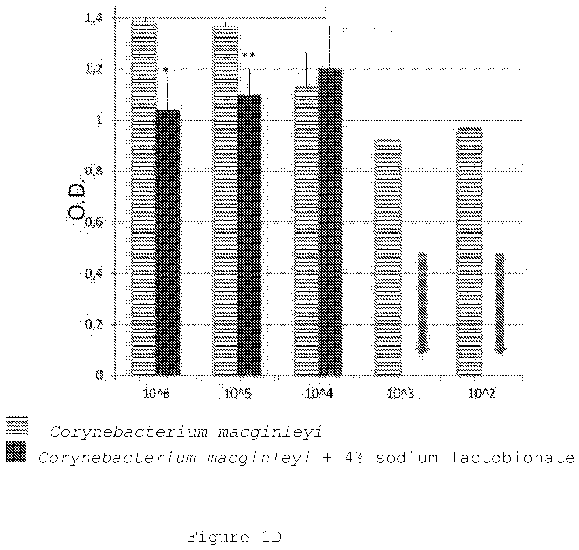

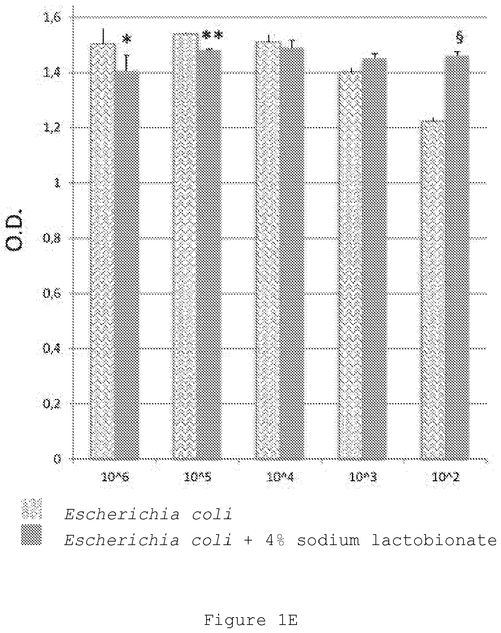

The results obtained from each bacterial species are graphically shown in Figs. 1A-E. Results have shown that a composition comprising 4% sodium lactobionate exerts a marked antibiotic action towards ocular and conjunctiva specific pathogens species, whereas has a more modest, or no, antimicrobial activity against other tested bacterial species.

Values represent means ± SD, *p<0.01 vs CTRL (One-way ANOVA, followed by Tukey's test)

### Comparison of in vitro antimicrobial activity of the composition according to the invention comprising sodium lactobionate and the association of sodium lactobionate and hyaluronic acid

Bacterial culture of *Staphylococcus aureus* was grown for 24 hours in MSA (Mannitol Salt Agar). The bacteria were suspended in a sterile physiological saline solution and bacterial cell density was assessed by optical density (OD) at 620 nm (Biotek Instruments, Elx-800). Final bacterial amount was standardized to 1x10⁶ CFU/ml. In order to investigate the antimicrobial activity, the microdilution method was used. Ten microliters (1x10⁶ CFU/ml) of bacterial suspension were seeded on 96 micro-well plate containing Muller-Hinton Broth (MHB, Oxoid - Thermo Scientific) and treatments were performed: 4% sodium lactobionate (LS) and 0.15% hyaluronic acid (HA) alone and in combination and their 2-fold serial dilutions in 100 µL final volume. Positive controls with bacterial suspension in nutrition broth and negative controls without bacterial suspension, were performed. Three replicates were designed of each test. Cell growth was measured using microplates reader at 620 nm. Microplate was mixed on microplate shaker (Biotek Instruments, Elx-800) with continuous shake for 24 hours at constant temperature of 37 °C.

Assayed compositions are summarized in Table 1. (compositions comprising 1% or 2% lactobionic acid are reference examples)

**Table 1**

| | |
|---|---|
| sodium lactobionate | 1% |
| sodium lactobionate | 2% |
| sodium lactobionate | 4% |
| hyaluronic acid | 0,037% |
| hyaluronic acid | 0,075% |
| hyaluronic acid | 0,15% |
| sodium lactobionate | 1% |
| hyaluronic acid | 0,037% |
| sodium lactobionate | 2% |
| hyaluronic acid | 0,75% |
| sodium lactobionate | 4% |
| hyaluronic acid | 0,15% |

### Results

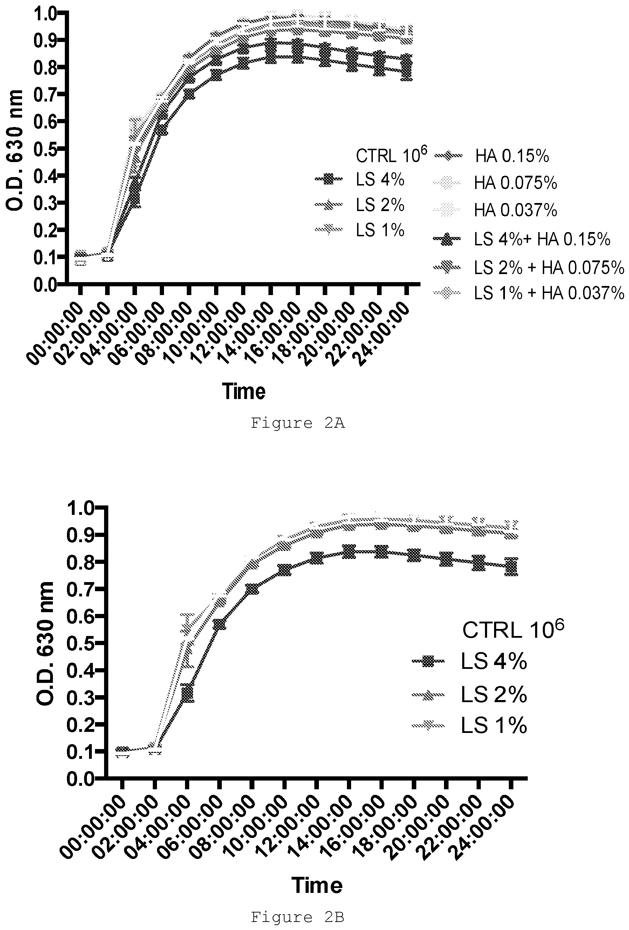

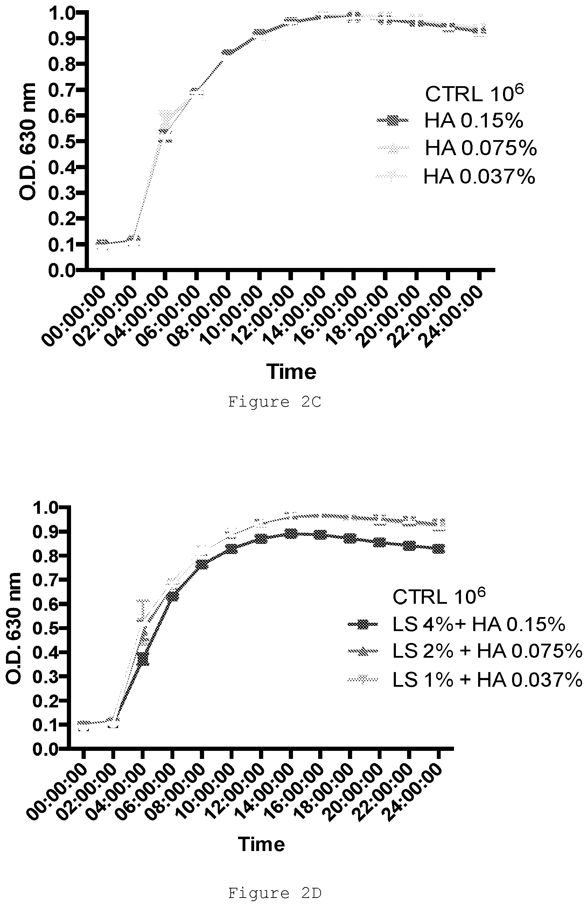

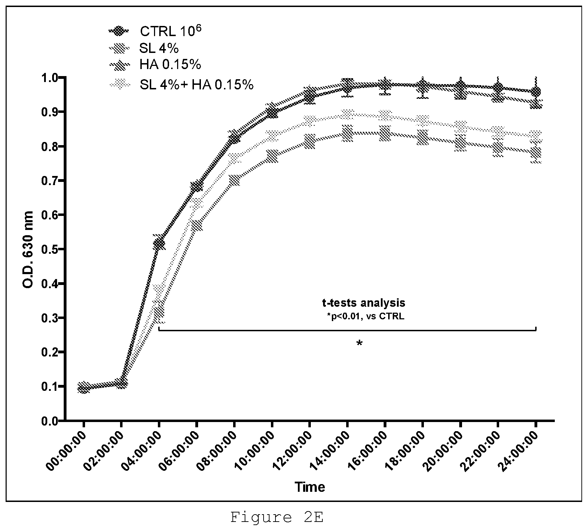

The results showed that composition comprising 4% sodium lactobionate has a modest antimicrobial activity. 4% sodium lactobionate comprising composition inhibited cell growth by 17% compared to control bacterial colture (CTRL), while composition comprising 0.15% hyaluronic acid showed a growth curve, comparable to CTRL. The presence of 0.15% hyaluronic acid in solution with 4% sodium lactobionate decreased the antimicrobial effect of 4% sodium lactobionate; in fact, the association of 4% sodium lactobionate and 0.15% hyaluronic acid inhibited the survival by 10% compared to CTRL (Figs. 2A-D). Other tested concentrations either for sodium lactobionate, for hyaluronic acid and their association, resulted comparable with CTRL.

Values represent means ± SD, *p<0.01 vs CTRL (One-way ANOVA, followed by Tukey's test).

### Conclusions

The *in vitro* study has clearly demonstrated the antimicrobial activity of a composition comprising sodium lactobionate and of a composition comprising the association of sodium lactobionate and hyaluronic acid which suggests the possible application for the use in the treatment of bacterial infections, and in particular of ophthalmic infections. The presence of hyaluronic acid which apparently decreases the antimicrobial performance of the composition (17% vs. 10% of bacterial growth inhibition), indeed exerts a beneficial effect keeping the eye hydrated and preserving from the onset of the well-known complications which could delay the healing process.

## Claims

1. A composition comprising 4% w/w lactobionic acid, or a salt thereof, and one or more pharmaceutically acceptable excipients and carriers for the use in the treatment of bacterial eye infections.

2. Composition for the use according to claim 1 further comprising hyaluronic acid, or a salt thereof, ranging between 0.01% w/w and 1.0% w/w.

3. Composition for the use according to claims 1 and 2, wherein the lactobionic acid, or its salt, is equal to 4% w/w, and hyaluronic acid, or its salt, is equal to 0.15% w/w.

4. Composition for the use according to any one of the preceding claims wherein the salt is a sodium salt.

5. Composition for the use according to any one of the preceding claims **characterized in that** it is isotonic, with osmolality between 250 and 350 mOsm/Kg and having pH between 7.0 and 7.4.

6. Composition for the use according to any one of the preceding claims **characterized in that** it further comprises stabilizing agents of the solution, lubricating agents, moisturizing agents, buffering agents, preservatives and chelating agents.

7. Composition for the use according to claim 6 comprising disodium EDTA and N-hydroxymethylglycinate.

8. Composition for the use according to any one of the preceding claims further comprising additional therapeutic agents, selected from the group of: growth factors, anti-inflammatory agents, vitamins, fibronectin, collagen and amino acids.

9. Composition for the use according to any one of the preceding claims in any form for topical application.

10. Composition for the use according to claim 9 in the form of collirium, gel, ointment, spray, eye drops.

11. Composition for the use according to any one of the preceding claims having the following formulation:
| | |
|---|---|
| Lactobionate sodium | 4.0% w/w |
| Sodium dihydrogen phosphate dihydrate | 0.225% w/w |
| Disodium phosphate dihydrate | 0.686% w/w |
| Disodium EDTA | 0.100% w/w |
| Sodium Hydroxymethylglycinate | 0.002% w/w |
| distilled water | q.s. to 100 g. |

12. Composition for the use according to any one of the preceding claims having the following formulation:
| | |
|---|---|
| Lactobionate sodium | 4.0% w/w |
| Sodium hyaluronate | 0.15% w/w |
| Sodium dihydrogen phosphate dihydrate | 0.225% w/w |
| Disodium phosphate dihydrate | 0.686% w/w |
| Disodium EDTA | 0.100% w/w |
| Sodium Hydroxymethylglycinate | 0.002% w/w |
| distilled water | q.s. to 100 g. |

13. Composition for the use according to any one of the preceding claims having the following formulation:
| | |
|---|---|
| Lactobionate sodium | 4.0% w/w |
| Sodium dihydrogen phosphate dihydrate | 0.225% w/w |
| Disodium phosphate dihydrate | 0.686% w/w |
| distilled water | q.s. to 100 g. |

14. Composition for the use according to any one of the preceding claims having the following formulation:
| | |
|---|---|
| Lactobionate sodium | 4.0% w/w |
| Sodium hyaluronate | 0.15% w/w |
| Sodium dihydrogen phosphate dihydrate | 0.225% w/w |
| Disodium phosphate dihydrate | 0.686% w/w |
| distilled water | q.s. to 100 g. |

15. Composition for the use according to claim 1 wherein the eye infection is: conjunctivitis, keratitis, blepharitis, blepharoconjunctivitis, orbital and preseptal cellulitis and endophthahnitis.

16. Eye pad or ocular bandage impregnated with a composition comprising 4.0% w/w lactobionic acid, or a salt thereof.

17. Eye pad or ocular bandage according to claim 16 impregnated with a composition further comprising hyaluronic acid, or a salt thereof, ranging between 0.01% w/w and 1.0% w/w.

## Patentansprüche

1. Zusammensetzung, umfassend 4 Gew.-% Lactobionsäure oder ein Salz davon, und ein oder mehrere pharmazeutisch annehmbare Arzneimittelträger und Trägersubstanzen zur Verwendung bei der Behandlung bakterieller Augeninfektionen.

2. Zusammensetzung zur Verwendung nach Anspruch 1, des Weiteren umfassend Hyaluronsäure oder ein Salz davon im Bereich von 0,01 Gew.-% und 1,0 Gew.-%.

3. Zusammensetzung zur Verwendung nach Ansprüchen 1 und 2, wobei die Lactobionsäure oder ihr Salz 4 Gew.-% entspricht, und Hyaluronsäure oder ihr Salz 0,15 Gew.-% entspricht.

4. Zusammensetzung zur Verwendung nach einem der vorhergehenden Ansprüche, wobei das Salz ein Natriumsalz ist.

5. Zusammensetzung zur Verwendung nach einem der vorhergehenden Ansprüche, **dadurch gekennzeichnet, dass** sie isotonisch ist mit einer Osmolalität zwischen 250 und 350 mOsm/kg und einen pH-Wert zwischen 7,0 und 7,4 besitzt.

6. Zusammensetzung zur Verwendung nach einem der vorhergehenden Ansprüche, **dadurch gekennzeichnet, dass** sie des Weiteren stabilisierende Wirkstoffe der Lösung, Gleitmittel, Feuchthaltemittel, Pufferwirkstoffe, Konservierungsmittel und Komplexbildner umfasst.

7. Zusammensetzung zur Verwendung nach Anspruch 6, umfassend Dinatrium-EDTA (Ethylendiamintetraessigsäure) und N-Hydroxymethylglycinat.

8. Zusammensetzung zur Verwendung nach einem der vorhergehenden Ansprüche, des Weiteren umfassend zusätzliche therapeutische Wirkstoffe, die ausgewählt sind aus der Gruppe: Wachstumsfaktoren, entzündungshemmende Mittel, Vitamine, Fibronectin, Collagen und Aminosäuren.

9. Zusammensetzung zur Verwendung nach einem der vorhergehenden Ansprüche in beliebiger Form zur lokalen Anwendung.

10. Zusammensetzung zur Verwendung nach Anspruch 9, in Form von Augentropfen, Gel, Salbe, Spray, Augentropfen.

11. Zusammensetzung zur Verwendung nach einem der vorhergehenden Ansprüche mit der folgenden Zubereitung:
| | |
|---|---|
| Lactobionatnatrium | 4,0 Gew.-% |
| Natriumdihydrogenphosphatdihydrat | 0,225 Gew.-% |
| Dinatriumphosphatdihydrat | 0,686 Gew.-% |
| Dinatrium EDTA | 0,100 Gew.-% |
| Natriumhydroxymethylglycinat | 0,002 Gew.-% |
| destilliertes Wasser | so viel wie nötig, bis 100 g |

12. Zusammensetzung zur Verwendung nach einem der vorhergehenden Ansprüche mit der folgenden Zubereitung:
| | |
|---|---|
| Lactobionatnatrium | 4,0 Gew.-% |
| Natriumhyaluronat | 0,15 Gew.-% |
| Natriumdihydrogenphosphatdihydrat | 0,225 Gew.-% |
| Dinatriumphosphatdihydrat | 0,686 Gew.-% |
| Dinatrium EDTA | 0,100 Gew.-% |
| Natriumhydroxymethylglycinat | 0,002 Gew.-% |
| destilliertes Wasser | so viel wie nötig, bis 100 g |

13. Zusammensetzung zur Verwendung nach einem der vorhergehenden Ansprüche mit der folgenden Zubereitung:
| | |
|---|---|
| Lactobionatnatrium | 4,0 Gew.-% |
| Natriumdihydrogenphosphatdihydrat | 0,225 Gew.-% |
| Dinatriumphosphatdihydrat | 0,686 Gew.-% |
| destilliertes Wasser | so viel wie nötig, bis 100 g |

14. Zusammensetzung zur Verwendung nach einem der vorhergehenden Ansprüche mit der folgenden Zubereitung:
| | |
|---|---|
| Lactobionatnatrium | 4,0 Gew.-% |
| Natriumhyaluronat | 0,15 Gew.-% |
| Natriumdihydrogenphosphatdihydrat | 0,225 Gew.-% |
| Dinatriumphosphatdihydrat | 0,686 Gew.-% |
| destilliertes Wasser | so viel wie nötig, bis 100 g |

15. Zusammensetzung zur Verwendung nach Anspruch 1, wobei die Augeninfektion: Bindehautentzündung, Hornhautentzündung, Augenlidentzündung, Augenlid- und Bindehautentzündung, Orbita- und präseptale Zellulitis und Endophthahnitis ist.

16. Augenpflaster oder Augenbinde, imprägniert mit einer Zusammensetzung, die 4,0 Gew.-% Lactobionsäure oder ein Salz davon enthält.

17. Augenpflaster oder Augenbinde nach Anspruch 16, imprägniert mit einer Zusammensetzung, die des Weiteren Hyaluronsäure oder ein Salz davon im Bereich zwischen 0,01 Gew.-% und 1,0 Gew.-% enthält.

## Revendications

1. Composition comprenant 4 % p/p d'acide lactobionique, ou un sel de celui-ci, et un ou plusieurs excipients et supports pharmaceutiquement acceptables pour l'utilisation dans le traitement d'infections oculaires bactériennes.

2. Composition pour utilisation selon la revendication 1, comprenant en outre de l'acide hyaluronique, ou un sel de celui-ci, se situant entre 0,01 % p/p et 1,0 % p/p.

3. Composition pour utilisation selon les revendications 1 et 2, dans laquelle l'acide lactobionique, ou son sel, est égal à 4 % p/p, et l'acide hyaluronique, ou son sel, est égal à 0,15 % p/p.

4. Composition pour utilisation selon l'une quelconque des revendications précédentes, dans laquelle le sel est un sel de sodium.

5. Composition pour utilisation selon l'une quelconque des revendications précédentes, **caractérisée en ce qu'**elle est isotonique, avec une osmolalité entre 250 et 350 mOsm/Kg et ayant un pH entre 7,0 et 7,4.

6. Composition pour utilisation selon l'une quelconque des revendications précédentes, **caractérisée en ce qu'**elle comprend en outre des agents stabilisants de la solution, des agents lubrifiants, des agents hydratants, des agents tampons, des conservateurs et des agents chélatants.

7. Composition pour utilisation selon la revendication 6, comprenant de l'EDTA dissodique et du N-hydroxyméthylglycinate.

8. Composition pour utilisation selon l'une quelconque des revendications précédentes, comprenant en outre des agents thérapeutiques supplémentaires, sélectionnés dans le groupe : des facteurs de croissance, des agents anti-inflammatoires, des vitamines, de la fibronectine, du collagène et des acides aminés.

9. Composition pour utilisation selon l'une quelconque des revendications précédentes, sous une forme quelconque pour une application topique.

10. Composition pour utilisation selon la revendication 9, sous la forme d'un collyre, d'un gel, d'un onguent, d'un aérosol, de gouttes ophtalmiques.

11. Composition pour utilisation selon l'une quelconque des revendications précédentes, ayant la formulation suivante :
| | |
|---|---|
| Lactobionate de sodium | 4,0 % p/p |
| Dihydrogénophosphate de sodium dihydraté | 0,225 % p/p |
| Phosphate dissodique dihydraté | 0,686 % p/p |
| EDTA dissodique | 0,100 % p/p |
| Hydroxyméthylglycinate de sodium | 0,002 % p/p |
| Eau distillée | q. s. à 100 g. |

12. Composition pour utilisation selon l'une quelconque des revendications précédentes, ayant la formulation suivante :
| | |
|---|---|
| Lactobionate de sodium | 4,0 % p/p |
| Hyaluronate de sodium | 0,15 % p/p |
| Dihydrogénophosphate de sodium dihydraté | 0,225 % p/p |
| Phosphate dissodique dihydraté | 0,686 % p/p |
| EDTA dissodique | 0,100 % p/p |
| Hydroxyméthylglycinate de sodium | 0,002 % p/p |
| Eau distillée | q. s. à 100 g. |

13. Composition pour utilisation selon l'une quelconque des revendications précédentes ayant la formulation suivante :
| | |
|---|---|
| Lactobionate de sodium | 4,0 % p/p |
| Dihydrogénophosphate de sodium dihydraté | 0,225 % p/p |
| Phosphate dissodique dihydraté | 0,686 % p/p |
| Eau distillée | q. s. à 100 g. |

14. Composition pour utilisation selon l'une quelconque des revendications précédentes ayant la formulation suivante :
| | |
|---|---|
| Lactobionate de sodium | 4,0 % p/p |
| Hyaluronate de sodium | 0,15 % p/p |
| Dihydrogénophosphate de sodium dihydraté | 0,225 % p/p |
| Phosphate dissodique dihydraté | 0,686 % p/p |
| Eau distillée | q. s. à 100 g. |

15. Composition pour utilisation selon la revendication 1, dans laquelle l'infection oculaire est: une conjonctivite, une kératite, une blépharite, une blépharoconjonctivite, une cellulite orbitaire et préseptale et une endophtalmie.

16. Compresse oculaire ou pansement oculaire imprégné(e) d'une composition comprenant 4,0 % p/p d'acide lactobionique, ou d'un sel de celui-ci.

17. Compresse oculaire ou pansement oculaire selon la revendication 16, imprégné(e) d'une composition comprenant en outre de l'acide hyaluronique, ou un sel de celui-ci, se situant entre 0,01 % p/p et 1,0 % p/p.
